# FASCICULE DE BREVET EUROPEEN

(11) **EP 3 928 518 B1**
(45) Date de publication et mention de la délivrance du brevet: **08.01.2025**
(21) Numéro de dépôt: 20709136.4
(22) Date de dépôt: 20.02.2020
(51) Int. Cl.: H04N 19/467, H04N 19/48, H04N 19/132, H04N 19/159, G06T 1/00, H04N 19/11, H04N 19/18

(54) **PROCÉDÉ PERMETTANT DE DISSIMULER DES DONNÉES DANS UNE IMAGE OU UN FLUX VIDÉO À L'INTÉRIEUR D'UNE CHAÎNE DE COMPRESSION**
VERFAHREN ZUM VERBERGEN VON DATEN IN EINEM BILD ODER EINEM VIDEOSTROM INNERHALB EINER KOMPRESSIONSKETT
METHOD FOR CONCEALING DATA IN AN IMAGE OR A VIDEO STREAM INSIDE A COMPRESSION CHAIN

(30) Priorité: 21.02.2019 FR 1901743
(43) Date de publication de la demande: 29.12.2021
(73) Titulaire: Université de Lorraine, 54000 Nancy (FR); Université de Strasbourg, 67000 Strasbourg (FR)
(72) Inventeur: MOUREAUX, Jean-Marie, 54130 SAINT-MAX (FR); CHAABOUNI, Amine, 54000 NANCY (FR); GAUDEAU, Yann, 67207 NIEDERHAUSBERGEN (FR)
(74) Mandataire: IPAZ
(86) Numéro de dépôt international: PCT/EP2020/054566
(87) Numéro de publication internationale: WO 2020/169781

(56) Documents cités:
- YUNXIA LIU ET AL: "Reversible Data Hiding Scheme Based On H.264/AVC without Distortion Drift", JOURNAL OF SOFTWARE, vol. 7, no. 5, 27 April 2012 (2012-04-27), XP055629600, ISSN: 1796-217X, DOI: 10.4304/jsw.7.5.1059-1065
- YIH-KAI LIN ED - CHUNG LAWRENCE ET AL: "High capacity reversible data hiding scheme based upon discrete cosine transformation", JOURNAL OF SYSTEMS & SOFTWARE, ELSEVIER NORTH HOLLAND, NEW YORK, NY, US, vol. 85, no. 10, 8 May 2012 (2012-05-08), pages 2395 - 2404, XP028401184, ISSN: 0164-1212, [retrieved on 20120606], DOI: 10.1016/J.JSS.2012.05.032
- LIUJUAN CAO ET AL: "Perception-driven watermarking with evolutionary block mapping", VISUAL COMMUNICATIONS AND IMAGE PROCESSING; 11-7-2010 - 14-7-2010; HUANG SHAN, AN HUI, CHINA,, 11 July 2010 (2010-07-11), XP030082218

## Description

La présente invention se rapporte à un procédé permettant de dissimuler des données dans une image ou un flux vidéo à l'intérieur d'une chaîne de compression.

Elle trouve une application particulièrement intéressante dans le domaine des vidéos médicales ORL et de la télémédecine, mais elle est d'un cadre plus large car elle peut s'appliquer à tout domaine dans lequel une chaîne de compression est utilisée la mise en place de blocs tels que décrit ci-après.

La dissimulation de données, connue aussi sous le nom de «data hiding», est une technique qui permet de cacher une information, sous forme d'une marque ou d'un message, dans un document numérique. Différents types d'information peuvent être cachés comme du texte, une signature, un code, une image, une vidéo ou de l'audio.

Cette technique, récente, a été initialement conçue, dans les années 90, pour lutter contre le piratage des données numériques et protéger la propriété intellectuelle des copies à l'identique, surtout après l'explosion de l'utilisation du support numérique durant la transmission et le stockage via des réseaux d'information très ouverts et sensibles aux attaques volontaires ou/et involontaires.

De nos jours, de nouveaux enjeux ont été découverts à travers différentes études et applications.

En fait, en plus du « copyright » et de l'authentification, le « data hiding » peut, aussi, être utilisé pour le transport de métadonnées, la correction d'erreur ou la réduction de la redondance de l'information, utile à l'amélioration des performances des systèmes de codage.

Depuis sa création, la dissimulation de l'information est développée dans plusieurs domaines comme par exemple les télécommunications, le codage des images/vidéos, les services VOD (Video On Demand), l'imagerie médicale et la sécurité informatique.

Les techniques de « data hiding » peuvent être classées en plusieurs catégories différentes :
- le canal caché : il s'agit d'un canal de communication, établi entre deux ordinateurs, qui utilise la bande passante d'un autre canal de manière invisible afin de transmettre les données sans la connaissance de l'administrateur du réseau. Cette notion est très sensible dans le contexte de la sécurité réseau.
- la stéganographie : c'est la dissimulation d'un ou de plusieurs messages dans un autre message de manière invisible sans pouvoir le détecter. Elle se distingue de la cryptographie qui vise plutôt à protéger un message en le rendant incompréhensible pour les personnes interdites à l'utiliser pour des motifs de sécurité comme la protection de la confidentialité, l'authenticité et l'intégrité des données. La stéganographie peut être utilisée avec du texte en cachant le message dans des lettres (stéganographie linguistique) ou dans une phrase, ou dans un support média (stéganographie technique) comme l'audio ou l'image. Elle a beaucoup été utilisée durant la deuxième guerre mondiale.
- l'anonymisation : cela consiste à éviter de mentionner le nom ou de publier les données privées d'une personne pour le respect de sa vie privée.
- le « Copyright marking» ou le marquage des droits d'auteur : c'est la dissimulation de l'information dans un document pour protéger les droits d'auteur. Par rapport à l'état de l'art de la stéganographie, les techniques du « Copyright » peuvent être visibles ou invisibles. Dans ce contexte, le « watermarking » (ou le tatouage numérique) et l'empreinte digitale sont les deux techniques les plus connues qui sont basées sur cette notion de dissimulation des données.

Pour chaque application, les principales contraintes à respecter sont : Capacité, Imperceptibilité/Invisibilité et Robustesse.

Il existe différentes méthodes de dissimulation de données dans l'image et la vidéo par différentes caractéristiques. On peut par exemple dissimuler de l'information selon :
- le domaine d'insertion : spatial, fréquentiel (ou spectral), multi-résolution ou fondé sur le contenu.
- le mode d'insertion de la marque : schéma additif, schéma substitutif.

Dans l'art antérieur, il est connu de réaliser la dissimulation dans la chaîne de compression.

On connaît le document Y. Liu, Z. Li et X. Ma, « Reversible Data Hiding Scheme Based On H.264/AVC without Distortion Drift », Journal of Software, vol. 7, avril 2012, décrivant une technique pour dissimuler des bits directement dans le module de transformation et quantification.

On connaît le document US6621933B2 qui décrit une méthode de tatouage appliquée au standard de compression MPEG2. Le tatouage se fait dans la chaîne de compression entre la transformation et la quantification.

On connaît le document US7006631B1 qui décrit une méthode pour cacher un tatouage numérique en modifiant des codes du codeur entropique. La dissimulation est donc directement mise en place au sein du codeur entropique.

La présente invention a pour but une nouvelle méthode permettant de dissimuler un maximum de données tout en préservant la qualité visuelle.

Un autre objet de l'invention est de solutionner le problème de bas débit dans des réseaux de capacité limitée.

L'invention est définie dans l'ensemble des revendications annexées.

L'invention peut s'appliquer à une image ou à un flux vidéo. Par bloc, on entend un bloc tel que défini dans la norme HEVC (autrement appelée H.265) ou un macrobloc tel que défini dans la norme AVC (autrement appelée H.264) ou tout bloc généré par une transformation de type DCT par exemple.

Avec le procédé selon l'invention, des données sont dissimulées en temps réel dans la chaîne de compression. L'application de ces données sur tout ou partie des blocs permet de choisir un compromis entre la qualité de l'image et la quantité de données dissimulées. Cela permet de rendre la vidéo ou l'image intelligente selon les besoins de l'utilisateur.

L'invention consiste en une méthode originale permettant de cacher le plus possible d'information dans une vidéo tout en gardant une qualité visuelle acceptable. Pour cela, une approche zonale a été utilisée pour la dissimulation des données dans des fréquences auxquelles l'oeil humain est peu sensible, tout en optimisant le temps de compression. Cela est grandement bénéfique dans certaines applications comme par exemple la transmission sur réseau de capteurs, à bas débit. On peut par exemple transférer le signal d'un électrocardiogramme en temps réel sans augmenter le débit de transmission ou faire de la télémedecine partout dans les régions équipées de connexions à faible débit. On lutte ainsi contre les déserts médicaux.

Le procédé selon l'invention permet ainsi de compresser et dissimuler, en même temps, différents types de données multimédias dans un support média type image ou vidéo. Les données peuvent être dissimulées pendant la compression, pendant une transmission en direct et/ou le stockage de la vidéo, sans augmenter la taille/le coût de transfert des données.

Avantageusement, la zone de hautes fréquences peut se situer sous la diagonale inférieure droite d'une matrice carrée d'un bloc.

La transformation peut être une transformation DCT (Discrète Cosine Transform), DST (Discrète Sine Transform) ou DWT (Discrète Wavelet Transform) utilisant des blocs de type TU pour « Transform Unit ».

D'une façon générale, suite à une transformation, telle que la transformation DCT (Discrète Cosine Transform) ou DST (Discrète Sine Transform), utilisée dans les derniers standards de compression comme H.264 et HEVC, l'information est répartie dans deux zones de fréquences différentes : la partie supérieure gauche, au-dessus de la diagonale d'un bloc DCT (les basses fréquences) et la partie inférieure droite, en-dessous de la diagonale (les moyennes et hautes fréquences).

L'oeil humain étant plus sensible aux basses fréquences qu'aux hautes, l'information importante de l'image est essentiellement traduite par les coefficients transformés de la zone supérieure. L'invention est ainsi astucieuse en ayant une approche en dissimulant les données dans les hautes fréquences, ce qui permet de limiter l'impact visuelle de la dégradation de la qualité de l'image du fait de l'insertion de données.

Cette approche zonale permet d'atteindre des capacités importantes allant jusqu'à 3 Mbits par exemple pour une séquence endoscopique ORL de seulement 10s compressée à 5 Mbits/s avec la norme HEVC.

Avantageusement, les données peuvent être dissimulées dans un flux vidéo, ces données constituant un flux dissimulé.

Le procédé selon l'invention permet par exemple de dissimuler un flux médical dans un autre flux pendant une session de télétransmission. Cela permet de réduire le nombre de flux médicaux à envoyer sur une plateforme donnée.

On répond ainsi aux contraintes de bas débit rencontrés notamment dans les réseaux du secteur médical. Dans la mesure où la transmission de différents flux à distance et en temps réel demande une bande passante importante, le fait de cacher un flux dans un autre permet de réduire le nombre de flux échangés entre les différents sites durant une session de consultation à distance par exemple.

Selon une caractéristique de l'invention, les données dissimulées peuvent constituer un tatouage numérique. Il s'agit d'un objet dissimulé pour une application de protection de droits d'auteur ou de données confidentielles.

Selon un mode de réalisation avantageux de l'invention, la dissimulation de données n'est réalisée que sur des blocs TU de taille prédéterminée. On peut par exemple limiter la dissimulation uniquement sur des blocs TU de taille 4x4 ou 8x8 ou 16x16 ou de préférence 32x32, cela dépend de la nature des images/vidéos traitées.

Selon l'invention, l'algorithme de la modification peut comprendre les étapes suivantes :
- si le bit à dissimuler est égal à 1, le coefficient transformé et quantifié doit être non nul, si le coefficient transformé et quantifié est nul avant la dissimulation, il est modifié à 1, sinon il garde sa valeur,
- si le bit à dissimuler est égal à 0, le coefficient transformé et quantifié doit être nul s'il était différent de 0 avant la dissimulation.

Malgré l'ajout des opérations dues à cet algorithme de dissimulation de données, il a été montré que le temps de traitement utilisé par la machine pendant la compression baisse légèrement par rapport à une compression sans dissimulation. Cette baisse peut être expliquée par l'augmentation du nombre de "0" après l'annulation des valeurs significatives, suite à l'application de l'algorithme d'insertion. Plus il y a de coefficients transformés quantifiés non significatifs (nuls), moins l'encodeur effectue de traitements pendant la compression. Ce résultat confirme la simplicité et l'efficacité de l'algorithme selon l'invention.

Ainsi, le procédé selon l'invention n'augmente pas la consommation de ressources CPU, quelques soit la quantité d'information insérée. Ce qui est compatible avec un fonctionnement temps réel.

Selon une caractéristique avantageuse de l'invention, pour un niveau k de dissimulation prédéfini, k étant compris entre 1 et N-1 pour un bloc TU de taille NxN, la modification s'applique uniquement sur des coefficients alignés selon une ligne parallèle à la diagonale, cette ligne étant la k^{ième} ligne depuis cette diagonale.

En d'autres termes, dans un bloc TU, il est possible de choisir d'appliquer la dissimulation que sur une partie de coefficients se trouvant sous la diagonale de façon à limiter la distorsion de l'image.

Selon une autre caractéristique avantageuse de l'invention, le procédé peut comprendre une étape d'application d'une saillance visuelle sur l'image traitée, la modification étant réalisée sur les blocs TU les plus saillants ou sur les blocs TU les moins saillants.

La saillance visuelle est une technique qui permet de définir les objets saillants dans une image, la zone de concentration des yeux durant la visualisation d'une vidéo, c'est à dire les objets possédant des caractéristiques perçues comme importantes par le système visuel. Cette zone saillante, présentant une information pertinente localisée spatialement dans l'image, peut être utile pour déterminer les régions d'intérêt R.O.I (« Région Of Interest ») dans une image.

En fonction du type de flux vidéo traité, il peut être avantageux de dissimuler les données uniquement dans les blocs TU des parties saillantes de l'image. Ce qui est le cas par exemple sur des vidéos endoscopiques ORL où la région d'intérêt est limitée. Mais il peut par ailleurs être avantageux de dissimuler les données uniquement dans les blocs TU des parties non saillantes de l'image. Ce qui est le cas par exemple sur des vidéos où les informations intéressantes sont réparties sur toute l'image.

Selon l'invention, les blocs TU les plus saillants peuvent être ceux qui présentent une valeur de saillance supérieure ou égale à un seuil prédéterminé compris entre 0 et 1.

Pour ce faire, on peut générer un fichier donnant un coefficient de saillance pour chaque bloc TU. Par la suite il est aisé d'appliquer un seuil de saillance égale à 0,4 par exemple (entre 0 et 1). Un bloc TU est alors dit saillant si la moyenne de tous ses coefficients transformés quantifiés est supérieure à 0,4. Dans ce cas on lui attribue une valeur égale à 1. Dans le cas contraire, on lui attribue un 0.

Selon un autre aspect de l'invention, il est proposé un encodeur de données configuré pour mettre en oeuvre le procédé décrit ci-dessus. A titre d'exemple, cet encodeur peut être configuré pour mettre en oeuvre la norme H.264 ou H.265. Précisément, les données peuvent être dissimulées selon l'invention après une transformation DCT/DST et une quantification, mais avant un codage entropique CABAC.

L'invention concerne également un décodeur de données configuré pour extraire les données dissimulées selon le procédé de l'invention.

Les données peuvent être extraites durant la lecture de la vidéo et peuvent être associées de façon à constituer un autre flux vidéo.

Il est également proposé un produit programme d'ordinateur comprenant des instructions qui, lorsque le programme est exécuté par un ordinateur, conduisent celui-ci à mettre en oeuvre les étapes du procédé selon l'invention.

D'autres avantages et caractéristiques de l'invention apparaîtront à l'examen de la description détaillée d'un mode de mise en oeuvre nullement limitatif, et des dessins annexés, sur lesquels :
[Fig. 1] est une vue schématique simplifiée d'une chaîne de compression ou encodeur selon l'invention,
[Fig. 2] est une vue schématique illustrant le procédé selon l'invention,
[Fig. 3] est une vue schématique d'un bloc TU 8x8 comprenant sept niveaux de coefficients transformés et quantifiés numérotés de un à sept,
[Fig. 4] est une vue schématique de trois modes lecture de blocs TU 4x4, et
[Fig. 5] est une vue illustrant une séquence vidéo endoscopique.

Les modes de réalisation qui seront décrits dans la suite ne sont nullement limitatifs; on pourra notamment mettre en oeuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier toutes les variantes et tous les modes de réalisation décrits sont prévus pour être combinés entre eux dans toutes les combinaisons où rien ne s'y oppose sur le plan technique.

Les différents modes de réalisation de la présente invention comprennent diverses étapes. Ces étapes peuvent être mises en oeuvre par des instructions d'une machine exécutable au moyen d'un microprocesseur par exemple.

Alternativement, ces étapes peuvent être réalisées par des circuits intégrés spécifiques comprenant une logique câblée pour exécuter les étapes, ou par toute combinaison de composants programmable et composants personnalisés.

La présente invention peut également être fournie sous forme d'un produit programme d'ordinateur qui peut comprendre un support mémoire informatique non-transitoire contenant des instructions exécutables sur une machine informatique, ces instructions pouvant être utilisées pour programmer un ordinateur (ou tout autre dispositif électronique) pour exécuter le procédé.

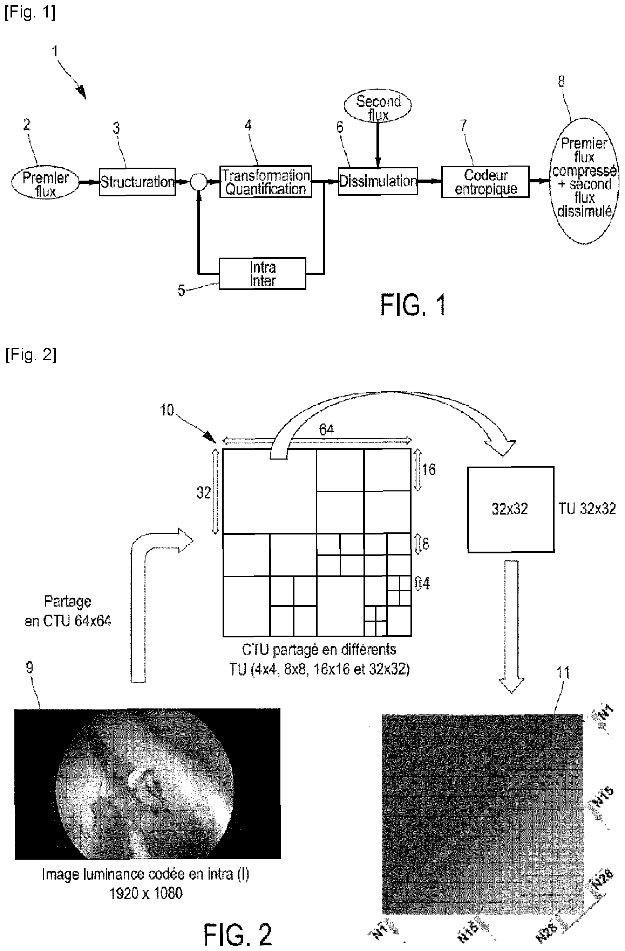

Sur la figure 1 on distingue globalement une chaîne de compression 1 recevant une séquence vidéo 2 en entrée. Cette chaîne fonctionne en boucle fermée dans la mesure où les données déjà codées sont disponibles pour traiter la partie courante. On parle de données causales qui sont obtenues par une boucle de décodage intégrée dans la chaîne. La vidéo en entrée est traitée image par image de façon séquentielle, chaque image étant divisée en slices représentant tout ou partie d'une image. Les slices sont eux-mêmes divisés en blocs dans la norme H.265 ou en macroblocs de taille 16×16 pixels dans la norme H.264, les macroblocs pouvant être partitionnés en rectangles de tailles plus petite. Les blocs ou macroblocs sont traités selon un parcours dit « raster », débutant en haut à gauche de l'image pour terminer en bas à droite.

Selon la norme H.265, chaque image subit une structuration de données dans le module 3, cela permet une séparation entre différents blocs, CTU (« Coding Tree Unit ») de tailles 16x16, 32x32 ou 64x64. Chaque CTU peut se partager en plus petits blocs, appelés CU (« Coding Unit »). On définit ainsi un arbre de partitionnement de codage, appelé « quadtree ». En fait, à partir de la racine, chaque noeud peut être divisé de manière récursive en quatre blocs de codage carrés CB (« Coding Block ») ayant la même taille. Cette différence permet d'avoir plus de performance au niveau du calcul. Ainsi, une zone homogène dans une image peut avoir des blocs de taille 64x64 et les régions hétérogènes ou les parties de vidéo avec beaucoup de mouvement peuvent se partager en blocs plus petits, 4x4 par exemple, afin d'apporter plus d'importance aux détails de la vidéo.

D'autres structures d'arbre de partitionnement sont imbriquées sur les feuilles du «quadtree» de codage. Ces subdivisions sont associées à la prédiction et au résiduel, appelé «quadtree» résiduel, avant d'appliquer la transformation fréquentielle et la quantification des coefficients résiduels.

Ainsi, chaque bloc CU peut se composer de plusieurs unités PU (« Prédiction Unit ») et TU (« Transform Unit ») en fonction du type de prédiction (Intra ou Inter) et du niveau de l'arbre «quadtree». Ces unités sont responsables du mode de décision de la prédiction et de l'application de la transformée dans chaque bloc CU.

A chaque bloc CU correspond à un bloc de luminance et 2 blocs de chrominance.

Les blocs TU subissent ensuite une transformée DCT et une quantification au sein du module 4. La boucle 5 comporte notamment une étape de prédiction permettant de réduire considérablement la quantité d'informations à transmettre, améliorant d'autant le niveau de compression. Il existe deux types de prédiction pixelliques, Intra et Inter, selon que l'on exploite respectivement les redondances spatiales ou bien temporelles. La prédiction Intra s'apparente au codage d'image fixe et exploite les données causales au sein de l'image courante et plus particulièrement voisines de l'ensemble de pixels courant. La prédiction Inter réalise une compensation de mouvement à partir d'une image précédemment encodée sur laquelle a été réalisée une estimation de mouvement. La nature des blocs traités dans le module de transformation et de quantification peut ainsi être Intra ou Inter. La présente invention cible spécifiquement les blocs Intra, et en particulier les blocs Intra de luminance.

La boucle 5 prélève donc les résiduels sortants du module de transformation et quantification 4 et réinjecte son résultat en entrée de ce module de transformation et quantification 4 via un comparateur recevant les images structurées.

Ainsi, chacun des blocs subit les étapes de prédiction générant un résiduel de pixels à partir des données causales, transformation et quantification du résiduel. Les résiduels sortant du module 4 sont des coefficients disposés dans des blocs TU. Selon l'invention, les blocs TU codés en Intra de la composante Luminance vont subir un traitement de dissimulation de données dans le module 6. Ces données peuvent être un marqueur numérique stocké en mémoire ou un second flux vidéo. Les blocs TU comportent alors des coefficients transformés, quantifiés et dissimulant des données selon l'invention. Ces blocs TU alimentent ensuite directement ou via un module de mise en forme non représenté un codeur entropique 7 de type CABAC (« Context-adaptive binary arithmetic coding »). La sortie est un flux compressé 8 incluant le second flux dont les bits sont dissimulés selon l'invention. Le codeur entropique reçoit généralement également des informations de signalisation nécessaires au décodage du flux. Le flux binaire obtenu en sortie de l'encodeur peut alors être lu par un décodeur qui restitue une vidéo en répétant les étapes de façon inverse.

La figure 1 a permet de visualiser clairement à quel endroit la fonction de dissimulation selon l'invention est réalisée, ce qui démarque l'invention d'une grande partie des techniques de l'art antérieur.

Sur la figure 2 on voit un peu plus en détail le processus de dissimulation selon l'invention. On distingue une image 9 qui est une image luminance codée en Intra avec une résolution 1920x1080.

La structuration permet d'obtenir un bloc CTU 10 de taille 64x64. Ce bloc est partagé en différents blocs TU de tailles 4x4, 8x8, 16x16 et 32x32. Selon un exemple de réalisation de l'invention, on sélectionne uniquement ou en plus d'autres blocs, le bloc TU 32x32 sur lequel va s'appliquer la dissimulation de données selon l'invention. On réalise une dissimulation de données dans les coefficients transformés quantifiés par approche zonale de niveau N1 au niveau N31 comme on le voit sur le bloc 11 de la figure 2.

On peut défini un niveau 1 de dissimulation permettant de cacher les données dans les 31 lignes de coefficients, parallèles à la diagonale et situées dans le coin bas à droite : de N1 à N31. Il également possible de définir un autre niveau 15 permettant de modifier les 17 lignes de N15 à N31.

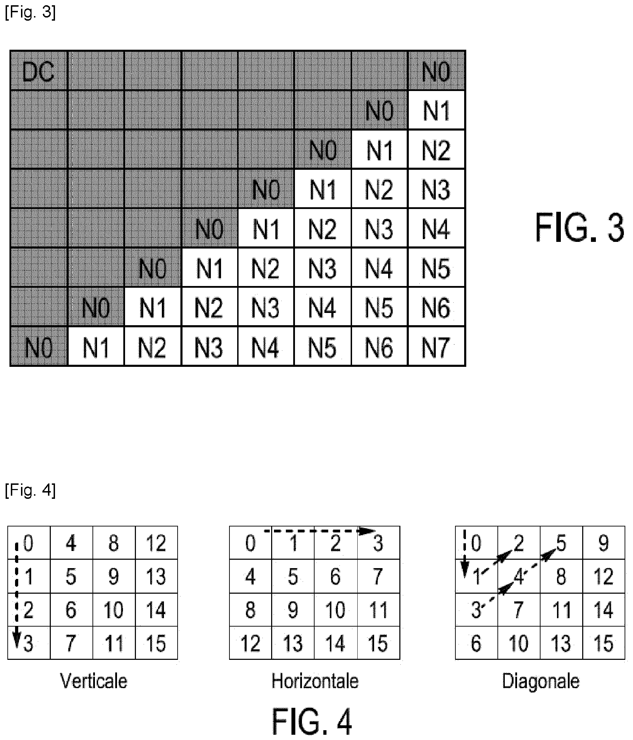

Sur la figure 3 est illustré un bloc TU 8x8 illustrant les 7 Niveaux de coefficients transformés et quantifiés numérotés de 1 à 7. Dans cet exemple, pour un bloc 8x8 (N=8), on peut définir 7 (N-1) niveaux par rapport à la diagonale, dans lesquels, on peut modifier les coefficients transformés et quantifiés. Les coefficients relatifs aux basses fréquences ne sont pas représentés. La diagonale est représentée de façon schématique avec la référence N0. Les coefficients de hautes fréquences sont représentés schématiquement avec les références Ni, i allant de 1 à 7. Pour des raisons de simplicité et d'identification des lignes parallèles à la diagonale, les coefficients de la même ligne comporte la même référence Ni.

Pour un niveau k choisi, on peut dissimuler le message dans tous les coefficients des N-k niveaux représentés. Ainsi, à chaque niveau, on peut modifier un pourcentage de coefficients dans le bloc TU. Comme le montre le tableau 3.1, dissimuler l'information au niveau 2 pour un bloc TU 8x8, correspond à modifier à peu près 33% des coefficients de ce bloc.

**[Tableaux 1]**

| Niveau | % de coefficients à modifier |
|---|---|
| 1 | 43,75 |
| 2 | 32,81 |
| 3 | 23,44 |
| 4 | 15,63 |
| 5 | 9,38 |
| 6 | 4,69 |
| 7 | 1,56 |

Le second tableau indique le pourcentage de coefficients à modifier pour dissimuler de l'information dans le cas d'un bloc TU 32x32.

**[Tableaux 2]**

| Niveau | % de coefficients à modifier | Niveau | % de coefficients à modifier |
|---|---|---|---|
| 1 | 48,44 | 17 | 11,72 |
| 2 | 45,41 | 18 | 10,25 |
| 3 | 42,48 | 19 | 8,89 |
| 4 | 39,65 | 20 | 7,62 |
| 5 | 36,91 | 21 | 6,45 |
| 6 | 34,28 | 22 | 5,37 |
| 7 | 31,74 | 23 | 4,39 |
| 8 | 29,30 | 24 | 3,52 |
| 9 | 26,95 | 25 | 2,73 |
| 10 | 24,71 | 26 | 2,05 |
| 11 | 22,56 | 27 | 1,46 |
| 12 | 20,51 | 28 | 0,98 |
| 13 | 18,55 | 29 | 0,59 |
| 14 | 16,70 | 30 | 0,29 |
| 15 | 14,94 | 31 | 0,10 |
| 16 | 13,28 | | |

Pour améliorer encore l'efficacité du procédé, il est prévu dans des cas particuliers, d'appliquer la saillance visuelle sur des vidéos médicales afin de ne sélectionner qu'une partie des blocs TU.

La saillance visuelle permet de définir les objets saillants dans une image, zones considérées comme affichant des éléments intéressants. Il est alors possible de choisir d'appliquer la dissimulation que sur des blocs faisant partie des zones saillantes ou des blocs faisant partie des zones non saillantes.

La saillance est traduite par une valeur allant de 0 à 1.

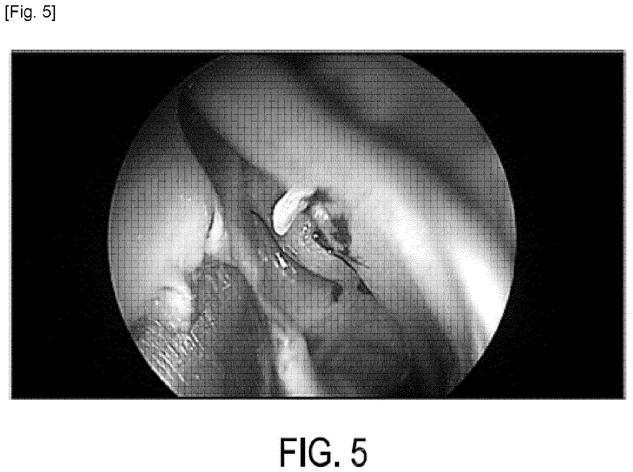

Par exemple, pour certaines images médicales de type ORL, comme l'image sur la figure 5, la zone utile est la partie centrale. Ainsi, en appliquant la technique de la saillance, la partie circulaire intérieure, qui représente l'information médicale, est plus saillante que la partie extérieure toute noire.

Les zones saillantes (partie circulaire intérieure) contiennent plus de coefficients significatifs que les zones non saillantes (partie noire).

Il est possible de générer un fichier donnant un coefficient de saillance pour chaque bloc TU. Par exemple, si on fixe un seuil de saillance égale à 0,4 (entre 0 et 1), un bloc TU est dit saillant si la moyenne de tous ses coefficients transformés quantifiés est supérieure à 0,4. Dans ce cas on lui attribue une valeur égale à 1. Dans le cas contraire, on lui attribue un 0.

En se basant sur ce fichier, seuls les blocs saillants peuvent être utilisés pour dissimuler les données. Une telle réalisation permet d'améliorer la dissimulation en capacité et en qualité.

Mais il peut être opportun de ne conserver que des blocs non saillants par exemple pour des images très chargées. Les coefficients significatifs se trouvent sur toute l'image et non pas sur une partie ou une zone spécifique.

Avec la présente invention, il est donc possible de sélectionner tout ou partie des blocs TU codés Intra de la composant de la luminance. Les blocs sélectionnés subissent alors un traitement de dissimulation consistant à modifier tout ou partie des coefficients de hautes fréquences de ces blocs.

Avantageusement, ce traitement de dissimulation est mis en oeuvre au moyen d'un algorithme de dissimulation selon l'invention de façon à insérer une grande quantité de données de manière simple. Cet algorithme peut être le suivant :

**[Tableaux 3]**

| Input | Coefficient transformé et quantifié C(i,j) de la composante luminance/ Bit dissimulé Tb | | | |
|---|---|---|---|---|
| Output | Coefficient après dissimulation Cₜ(i,j) | | | |
| 1 | | DEBUT | | |
| 2 | | Si Tb = 1 alors | | |
| 3 | | | Si C(i,j) = 0 alors | |
| 4 | | | | Cₜ(i,j) = 1 |
| 5 | | | Sinon Cₜ(i,j) = C(i,j) | |
| 6 | | | Fin | |
| 7 | | Sinon (Si Tb = 0) | | |
| 8 | | | | Cₜ(i,j) = 0 |
| 9 | | Fin | | |
| 10 | | FIN | | |

Si le bit à dissimuler Tb est égal à 1, le coefficient transformé et quantifié C(i,j) doit être non nul. S'il est nul avant la dissimulation, on le modifie à 1, sinon il garde sa valeur. Cette manipulation minimise la distorsion qu'on peut avoir suite à la dissimulation.

Dans le cas contraire (Tb=0), l'algorithme annule le coefficient C(i,j) s'il était différent de 0 avant la dissimulation.

Cet algorithme peut par exemple être intégré dans un encodeur temps réel x265.

Avant de cacher des données, le mode de lecture des coefficients pour chaque bloc TU peut être déterminé de façon à en tenir compte lors de la dissimulation. Ce mode de lecture dépend de la taille du bloc et du mode de prédiction appliqué pour générer les coefficients. Trois modes de lecture sont définis dans le standard HEVC :
- Lecture verticale : Taille TU 4x4 et 8x8 pour les prédictions intra {6, 7, 8, 9, 10, 12, 13, 14}
- Lecture horizontale : Taille TU 4x4 et 8x8 pour les prédictions intra {22, 23, 24, 25, 26, 27, 28, 29, 30}
- Lecture diagonale : Taille TU 4x4 et 8x8 pour les prédictions intra {0, 1, 2, 3, 4, 5, 15, 16, 17, 18, 19, 20, 21, 31, 32, 33, 34, 35} et Taille TU 16x16 et Taille TU 32x32 pour toutes les prédictions intra.

Ces trois modes de lecture sont présentés sur la figure 4 dans l'exemple d'un bloc TU 4x4.

La connaissance de mode de lecture est avantageuse pour le fonctionnement de l'algorithme au moment de l'insertion et l'extraction de message dissimulé. Ceci permet de vérifier si le coefficient en cours d'analyse est bien dans la zone sélectionnée avant d'appliquer la dissimulation. Cette vérification se fait par la position et l'ordre de chaque coefficient dans le bloc TU associé. Par exemple, un coefficient 12 dans un bloc 8x8 en mode de lecture verticale n'est pas égal au coefficient 12 dans un bloc 8x8 qui suit le mode de lecture horizontale. Le même principe pour chaque coefficient sauf le premier coefficient et le dernier coefficient du bloc.

En suivant les mêmes étapes que celles de l'insertion, l'algorithme d'extraction du message peut être le suivant :

**[Tableaux 4]**

| Input | Coefficient après dissimulation Cₜ(i,j) | |
|---|---|---|
| Output | Bit dissimulé Tb | |
| 1 | DEBUT | |
| 2 | Si Cₜ(i,j) = 0 alors | |
| 3 | | Tb = 0 |
| 4 | Sinon (Si Cₜ(i,j) <> 0) | |
| 5 | | Tb = 1 |
| 6 | Fin | |
| 7 | FIN | |

Ainsi, si le coefficient modifié Cₜ(i,j) est nul, le bit dissimulé Tb est nul. Sinon, il est égal à 1. Cet algorithme permet de simplifier le traitement au niveau du décodeur qui ne doit pas apporter de temps de traitement supplémentaire significatif durant le décodage de la vidéo.

Par exemple, pour des séquences vidéos médicales, avec un mode de lecture associé pour une compression x265, à un débit de 5 Mbits/s avec une configuration standard de 5 images I codées en intra pour 10s, les résultats sont affichés dans les deux tableaux suivants. Le tableau 5 illustre la répartition des blocs TU suivant le mode de lecture. Le tableau 6 illustre la répartition des blocs TU suivant leur taille.

**[Tableaux 5]**

| Mode de lecture du bloc TU | Séquence 2 (%) |
|---|---|
| Diagonal | 99,9 |
| Horizontal | 0,1 |
| Vertical | 0 |

**[Tableaux 6]**

| Taille du bloc TU | Séquence 2 (%) |
|---|---|
| 4x4 | 0,1 |
| 8x8 | 0,4 |
| 16x16 | 4,4 |
| 32x32 | 95,1 |

Ces résultats montrent que la majorité des blocs TU sont de grande taille 32x32 (95,1%) et avec une lecture diagonale (99,9%), pour ce type de vidéo médicale.

Pour simplifier le procédé selon l'invention, la dissimulation peut s'appliquer uniquement sur les blocs TU 32x32 avec le mode de lecture diagonale.

Par ailleurs, il a été constaté que malgré l'ajout des opérations dues à l'algorithme selon l'invention, le temps de traitement utilisé pendant la compression baisse légèrement par rapport à une compression sans dissimulation. Cette baisse peut être expliquée par l'augmentation du nombre de "0" après l'annulation des valeurs significatives, suite à l'application du procédé selon l'invention. Plus il y a des coefficients transformés quantifiés non significatifs (nuls), moins l'encodeur effectue de traitement pendant la compression. Ce résultat confirme la simplicité et l'efficacité du procédé de dissimulation selon l'invention. Ainsi, ce procédé n'augmente pas la consommation de ressources CPU, quelle que soit la quantité d'information insérée, ceci étant un paramètre avantageux pour les applications temps réel.

La présente invention est une nouvelle approche de dissimulation de données permettant le transport de métadonnées supplémentaires à coût constant. Pour ce faire, les données sont insérées dans les blocs TU après quantification et avant encodage entropique CABAC, plus précisément dans la zone inférieure droite par rapport à la diagonale des blocs TU codés en intra et associés à la composante de la luminance. Cette approche zonale permet d'atteindre des capacités importantes allant jusqu'à 3 Mbits pour une séquence endoscopique ORL de seulement 10s.

Par ailleurs, il est possible de définir différents niveaux d'insertion, ce qui rend la technique paramétrable et évolutif, permettant ainsi de gérer la capacité en fonction de la qualité.

La technique de saillance visuelle permet de réduire le nombre de blocs à traiter tout en améliorant la capacité (quantité de données dissimulées) et la qualité (aspect visuel).

## Revendications

1. Procédé permettant de dissimuler des données dans une image ou un flux vidéo à l'intérieur d'une chaîne de compression, ce procédé étant mis en oeuvre par ordinateur et comprenant les étapes suivantes :
- une phase de structuration et de traitement au cours de laquelle au moins une image est structurée en des blocs comportant des coefficients,
- une phase de transformation et de quantification des blocs de façon à générer des coefficients transformés et quantifiés,
- un codage entropique destiné à encoder les coefficients transformés et quantifiés,
**caractérisé en ce qu'**il comprend en outre une étape de dissimulation de données au cours de laquelle des bits desdites données sont dissimulés en modifiant des coefficients transformés et quantifiés situés dans une zone de hautes fréquences d'une partie au moins desdits blocs qui sont relatifs à la composante luminance du flux vidéo et qui sont codés en Intra,
**en ce que** cette modification est réalisée après la quantification mais avant le codage entropique ; et **en ce que** pour un niveau k de dissimulation prédéfini, k étant compris entre 1 et N-1 pour un bloc de taille NxN, la modification s'applique uniquement sur des coefficients alignés selon une ligne parallèle à la diagonale, cette ligne étant la k^{ième} ligne depuis cette diagonale.

2. Procédé selon la revendication 1, **caractérisé en ce que** la dissimulation de données n'est réalisée que sur des blocs de taille prédéterminée.

3. Procédé selon la revendication 1 ou 2, **caractérisé en ce que** la modification comprend les étapes suivantes : - si le bit à dissimuler est égal à 1, le coefficient transformé et quantifié doit être non nul, si le coefficient transformé et quantifié est nul avant la dissimulation, il est modifié à 1, sinon il garde sa valeur, - si le bit à dissimuler est égal à 0, le coefficient transformé et quantifié est égale à 0.

4. Procédé selon l'une quelconque des revendications précédentes, **caractérisé en ce que** la zone de hautes fréquences est la zone située sous la partie inférieure droite, en dessous de la diagonale d'une matrice carrée d'un bloc.

5. Procédé selon l'une quelconque des revendications précédentes, **caractérisé en ce qu'**il comprend une étape d'application d'une saillance visuelle sur l'image traitée, la modification étant réalisée sur les blocs les plus saillants ou sur les blocs les moins saillants.

6. Procédé selon la revendication 5, **caractérisé en ce que** les blocs les plus saillants sont ceux qui présentent une valeur de saillance supérieure ou égale à un seuil prédéterminé compris entre 0 et 1.

7. Procédé selon l'une quelconque des revendications précédentes, **caractérisé en ce que** les données sont dissimulées dans un flux vidéo, ces données constituant un flux dissimulé.

8. Procédé selon l'une quelconque des revendications 1 à 6, **caractérisé en ce que** les données dissimulées constituent un tatouage numérique.

9. Procédé selon l'une quelconque des revendications précédentes, **caractérisé en ce que** la transformation est une transformation DCT (Discrète Cosine Transform), DST (Discrète Sine Transform) ou DWT (Discrète Wavelet Transform) utilisant des blocs de type TU pour « Transform Unit ».

10. Encodeur de données configuré pour mettre en oeuvre le procédé selon la revendication 1.

11. Encodeur selon la revendication 10, **caractérisé en ce qu'**il est configuré pour mettre en oeuvre la norme H.264 ou H.265.

12. Produit programme d'ordinateur comprenant des instructions qui, lorsque le programme est exécuté par un ordinateur, conduisent celui-ci à mettre en oeuvre les étapes du procédé selon la revendication 1.

## Patentansprüche

1. Verfahren, das es ermöglicht, Daten in einem Bild oder einem Videostrom innerhalb einer Komprimierungskette zu verbergen, wobei dieses Verfahren durch einen Computer implementiert wird und die folgenden Schritte umfasst:
- eine Strukturierungs- und Verarbeitungsphase, während der mindestens ein Bild in Blöcke strukturiert wird, die Koeffizienten aufweisen,
- eine Phase zum Transformieren und Quantifizieren der Blöcke, um transformierte und quantifizierte Koeffizienten zu erzeugen,
- eine entropische Codierung, die dazu bestimmt ist, die transformierten und quantifizierten Koeffizienten zu codieren,
**dadurch gekennzeichnet, dass** es ferner einen Schritt zum Verbergen von Daten umfasst, während dessen Bits der Daten durch Modifizieren der transformierten und quantifizierten Koeffizienten verborgen werden, die sich in einer Hochfrequenzzone von mindestens einem Teil der Blöcke befinden, die sich auf die Luminanzkomponente des Videostroms beziehen und die intracodiert sind,
**dadurch, dass** diese Modifikation nach der Quantifizierung, aber vor der entropischen Codierung durchgeführt wird; und **dass** für einen vordefinierten Verbergungsgrad k, wobei k für einen Block von Größe NxN zwischen 1 und N-1 liegt, die Modifikation nur für Koeffizienten gilt, die entlang einer Linie parallel zu der Diagonale ausgerichtet sind, wobei diese Linie die k-te Linie von dieser Diagonale ist.

2. Verfahren nach Anspruch 1, **dadurch gekennzeichnet, dass** das Verbergen von Daten nur für Blöcke von vorbestimmter Größe durchgeführt wird.

3. Verfahren nach Anspruch 1 oder 2, **dadurch gekennzeichnet, dass** die Modifikation die folgenden Schritte umfasst:
- falls das zu verbergende Bit gleich 1 ist, der transformierte und quantifizierte Koeffizient ungleich null sein muss, falls der transformierte und quantifizierte Koeffizient vor dem Verbergen null ist, er auf 1 modifiziert wird, andernfalls seinen Wert beibehält,
- falls das zu verbergende Bit gleich 0 ist, der transformierte und quantifizierte Koeffizient gleich 0 ist.

4. Verfahren nach einem der vorstehenden Ansprüche,
**dadurch gekennzeichnet, dass** der Hochfrequenzbereich der Bereich ist, der sich unterhalb der Diagonale einer quadratischen Matrix eines Blocks unter dem unteren rechten Teil befindet.

5. Verfahren nach einem der vorstehenden Ansprüche,
**dadurch gekennzeichnet, dass** es einen Schritt zum Anwenden einer visuellen Hervorhebung auf das verarbeitete Bild umfasst, wobei die Modifikation an den am stärksten hervorgehobenen Blöcken oder an den am wenigsten hervorgehobenen Blöcken durchgeführt wird.

6. Verfahren nach Anspruch 5, **dadurch gekennzeichnet, dass** die am stärksten hervorgehobenen Blöcke diejenigen sind, deren Hervorhebungswert größer als oder gleich einem vorbestimmten Schwellenwert zwischen 0 und 1 ist.

7. Verfahren nach einem der vorstehenden Ansprüche,
**dadurch gekennzeichnet, dass** die Daten in einem Videostrom verborgen werden, wobei diese Daten einen verborgenen Strom darstellen.

8. Verfahren nach einem der Ansprüche 1 bis 6,
**dadurch gekennzeichnet, dass** die verborgenen Daten ein digitales Wasserzeichen darstellen.

9. Verfahren nach einem der vorstehenden Ansprüche,
**dadurch gekennzeichnet, dass** die Transformation eine DCT (Diskrete Kosinustransformation), DST (Diskrete Sinustransformation) oder DWT (Diskrete Wavelet-Transformation) ist, die Blöcke der Art TU für "Transformationseinheit" nutzt.

10. Datencodierer, der zum Implementieren des Verfahrens nach Anspruch 1 konfiguriert ist.

11. Codierer nach Anspruch 10, **dadurch gekennzeichnet, dass** er zum Implementieren des H.264- oder H.265-Standards konfiguriert ist.

12. Computerprogrammprodukt, umfassend Anweisungen, die, wenn das Programm durch einen Computer ausgeführt wird, diesen dazu veranlassen, die Schritte des Verfahrens nach Anspruch 1 zu implementieren.

## Claims

1. Method for concealing data in an image or a video stream inside a compression chain, this method being implemented by computer and comprising the following steps:
- a phase of structuring and processing during which at least one image is structured in blocks containing coefficients,
- a phase of transformation and quantization of the blocks so as to generate transformed and quantized coefficients,
- an entropy encoding intended to encode the transformed and quantized coefficients,
**characterized in that** it also comprises a data concealment step during which bits from said data are concealed by modifying transformed and quantized coefficients, situated in a zone of high frequencies of at least a part of said blocks which are relative to the luminance component of the video stream and which are intra-coded,
**in that** this modification is carried out after the quantization but before the entropy encoding;
and **in that** for a predefined concealment level k, k being comprised between 1 and N-1 for a block of size N x N, the modification applies only to the coefficients aligned on a row parallel to the diagonal, this row being the k^{th} row from this diagonal.

2. Method according to claim 1, **characterized in that** data concealment is only carried out on blocks of predetermined size.

3. Method according to claim 1 or 2, **characterized in that** the modification comprises the following steps:
- if the bit to be concealed is equal to 1, the transformed and quantized coefficient must be non-zero; if the transformed and quantized coefficient is zero before concealment, it is modified to 1, otherwise it retains its value,
- if the bit to be concealed is equal to 0, the transformed and quantized coefficient is equal to 0.

4. Method according to any one of the preceding claims, **characterized in that** the zone of high-frequencies is the zone situated under the lower right part, below a square matrix of a block.

5. Method according to any one of the preceding claims, **characterized in that** it comprises a step of applying a visual prominence to the processed image, the modification being carried out on the most prominent blocks or on the least prominent blocks.

6. Method according to claim 5, **characterized in that** the most prominent blocks are those having a prominence value greater than or equal to a predetermined threshold comprised between 0 and 1.

7. Method according to any one of the preceding claims, **characterized in that** the data are concealed in a video stream, these data constituting a concealed stream.

8. Method according to any one of claims 1 to 6, **characterized in that** the concealed data constitute a watermark.

9. Method according to any one of the preceding claims, **characterized in that** the transform is a discrete cosine transform (DCT), discrete sine transform (DST), or discrete wavelet transform (DWT) using blocks of the transform unit (TU) type.

10. Data encoder configured to implement the method according to claim 1.

11. Encoder according to claim 10, **characterized in that** it is configured to implement the H.264 or H.265 standard.

12. Computer program product comprising instructions which, when the program is executed by a computer, control the latter to implement the steps of the method according to claim 1.
